# FASCICULE DE BREVET EUROPEEN

(11) **EP 1 885 323 B1**
(45) Date de publication et mention de la délivrance du brevet: **15.07.2009**
(21) Numéro de dépôt: 06708321.2
(22) Date de dépôt: 16.02.2006
(51) Int. Cl.: A61K 8/64, A61Q 7/00

(54) **UTILISATION COSMÉTIQUE D'AU MOINS UN TÉTRAPEPTIDE NATUREL AC-N-SER-ASP-LYS-PRO- OU UN DE SES ANALOGUES EN TANT QU'AGENT POUR RALENTIR LA CHUTE DES CHEVEUX ET/OU STIMULER LEUR CROISSANCE**
KOSMETISCHE VERWENDUNG VON MINDESTENS EINEM NATÜRLICHEN AC-N-SER-ASP-LYS-PRO-TETRAPEPTID ODER EINEM SEINER ANALOGA ALS MITTEL ZUR VERLANGSAMUNG DES HAARAUSFALLS UND/ODER ZUR STIMULIERUNG DES HAARWACHSTUMS
COSMETIC USE OF AT LEAST ONE NATURAL AC-N-SER-ASP-LYS-PRO-TETRAPEPTIDE OR ONE OF ITS ANALOGUES AS AGENT FOR SLOWING DOWN LOSS OF HAIR AND/OR STIMULATING HAIR GROWTH

(30) Priorité: 18.02.2005 FR 0501672
(43) Date de publication de la demande: 13.02.2008
(73) Titulaire: Centre National de la Recherche Scientifique - CNRS, 75016 Paris Cedex 16 (FR)
(72) Inventeur: BAKALA, Joanna, Elzbieta, F-75012 Paris (FR); BIGNON, Jerôme, F-91530 Le Val Saint Germain (FR); LALLEMAND, Jean-Yves, F-91120 Palaiseau (FR)
(74) Mandataire: Ahner, Francis
(86) Numéro de dépôt international: PCT/EP2006/060029
(87) Numéro de publication internationale: WO 2006/087363

(56) Documents cités:
- WO-A-00/32620
- WO-A-02/24218
- WO-A-88/00594
- WO-A-20/05010027

## Description

La présente invention concerne l'utilisation cosmétique dans une composition, d'au moins un composé tétrapeptidique ou d'un analogue, en tant qu'agent pour ralentir la chute des cheveux et/ou stimuler leur croissance.

La chevelure humaine représente un ensemble d'environ 150 000 cheveux. Un cheveu est composé d'une tige, partie libre qui émerge à la surface du cuir chevelu, et d'une racine (bulbe), implantée dans un follicule pileux. La durée de vie d'un cheveu varie de 2 à 7 ans.

Les cheveux sont produits par un organe complexe, le follicule pileux, formé d'un compartiment dermique et d'un compartiment épithélial, chacun de ces compartiments étant subdivisé en sous-compartiments fonctionnels. Les follicules servent également de « réservoir » de cellules souches capables de donner naissance à toutes les lignées de cellules nécessaires pour reconstituer les follicules eux-mêmes, l'épiderme et les glandes sébacées.

Chez l'être humain, la croissance des cheveux et leur renouvellement sont principalement déterminés par l'activité des follicules pileux et par leur environnement dermo-épidermique. Un follicule est, soit en phase de croissance (production de la tige pilaire) au cours de laquelle les cheveux s'allongent (phase anagène), soit en arrêt de croissance (phase catagène), soit en phase de repos, phase durant laquelle se produit la chute du cheveu (phase télogène). A la suite de la phase télogène et par un processus de néo-morphogenèse, le follicule se régénère sur place à partir d'un réservoir de cellules souches et initie une nouvelle phase anagène. Sur une chevelure normale qui se renouvelle en permanence, environ 85 % des follicules sont en phase de croissance, 2 % en phase de repos et plus de 10 % en phase de chute.

Les hommes et les femmes perdent habituellement de 50 à 150 cheveux par jour. Ces cheveux sont toutefois immédiatement remplacés par de nouvelles pousses suivant le cycle de croissance pileux. Si la perte de cheveux devient visible, c'est qu'elle est excessive (dépassant de loin 150 cheveux par jour) ou, plus couramment, que les cheveux qui tombent sont remplacés par des poils plus fins, comme c'est le cas des personnes atteintes d'alopécie androgénétique. Dans un tel cas, les cheveux qui repoussent sont de plus en plus fins et se raréfient d'un cycle à l'autre.

Les causes de la perte de cheveux peuvent être diverses : maladies comme le cancer ou le lupus, modifications hormonales, stress, prise de certains médicaments ou carences alimentaires. Mais dans la très grande majorité des cas, la chute est d'origine héréditaire et hormonale. Chez la femme, la perte de cheveux frappe les sujets surtout après la ménopause.

En fonction de l'origine de la calvitie, les cheveux peuvent repousser plus
ou moins facilement. II n'existe pas de traitement passe-partout, chaque cas de chute de cheveux est complexe et doit faire l'objet d'un traitement approprié. Dans les cas d'une chute androgénétique, principale cause de calvitie tant chez l'homme que chez la femme, il faut un traitement rapide et efficace. Si rien n'est entrepris, on assiste à l'atrophie progressive des follicules pileux et des racines, à l'affinement du diamètre des cheveux et au développement d'une alopécie. II n'existe pas de traitement à visée curative contre l'alopécie androgénétique. En effet, elle résulte d'une trop grande sensibilité des follicules pileux à la dihydrotestostérone (DHT) qui abrège la phase de croissance capillaire. L'administration d'antiandrogènes oraux comme le Finastéride (Propecia®), qui bloque une enzyme responsable de la transformation de la testostérone en dihydrotestostérone au niveau des follicules pileux, réduit la chute des cheveux et active leur repousse. Cependant, ce produit qui était porteur de beaucoup d'espoir est contre indiqué chez la femme et de récentes mises en garde ont été annoncées quand à son utilisation chez l'homme. En effet, les résultats d'une étude clinique suggèrent qu'il existe un risque de développer une forme grave du cancer de la prostate chez les patients prenant ce médicament.

Le Minoxidil (Rogaine ®), une autre préparation anti-chute disponible actuellement sur le marché n'est utile qu'au début de l'alopécie. En effet, son application topique sur le cuir chevelu retarde la fin des cycles de croissance capillaire, donc la perte des cheveux, mais ne permet pas de reconstruire le follicule inactif. En cas d'arrêt du traitement, la chute reprend après quelques semaines.

Il existe également des produits cosmétiques contenant des agents propres à conférer du volume à la chevelure amincie. Ces agents ne stimulent cependant pas la pousse. Ils peuvent toutefois donner l'impression d'une chevelure plus fournie en recouvrant la tige du cheveu, augmentant ainsi son diamètre. Ces produits n'offrent qu'une solution temporaire puisqu'ils disparaissent après chaque shampooing.

Il serait donc très utile de mettre à la disposition de l'industrie cosmétique ou pharmaceutique un produit efficace et dépourvu d'effets secondaires, pour stimuler la croissance des cheveux et/ou empêcher leur chute. Ce produit permettrait donc de réduire, voire de supprimer l'alopécie.

La Demanderesse a mis en évidence qu'un tétrapeptide naturel ou ses analogues permettaient de stimuler des fonctions vitales du cheveu et en particulier d'activer les cellules souches épithéliales, et donc d'atteindre le but recherché. Ces dérivés présentent en outre l'avantage d'être obtenus par des voies de synthèse peptidiques faciles à mettre en oeuvre et donc peu coûteuses. De plus, ces composés présentent une toxicité très faible voire nulle vis-à-vis de l'organisme.

Les peptides ou analogues mis en oeuvre dans le cadre de la présente invention, sont dérivés de la structure de base Acétyl-Ser-Asp-Lys-Pro (AcSDKP). Ils sont connus pour leurs propriétés thérapeutiques (WO-88/00594 et WO-97/28183).

Récemment, la Demanderesse a mis en évidence leurs propriétés angiogéniques (WO-02/24218).

Aucun des documents de l'art antérieur ne décrit ni ne suggère que ces composés puissent avoir une action anti-chute au niveau des cheveux en cosmétique, ni que leur utilisation permette d'avoir un effet positif sur la stimulation des cellules souches présentes dans les follicules pileux.

La présente invention concerne donc l'utilisation cosmétique d'au moins un composé de formule (I) : 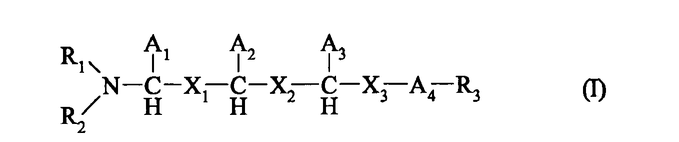 dans laquelle,
A₁ est le radical correspondant à D- ou L- Ser,
A₂ est le radical correspondant à D- ou L- Asp ou Glu,
A₃ est le radical correspondant à D- ou L- Lys, Arg ou Orn,
A₄ est le radical correspondant à D- ou L- Pro,
R₁ et R₂ sont choisis indépendamment parmi l'atome d'hydrogène, les groupements alkyle C₁-C₁₂ linéaire ou ramifié substitué ou non, arylalkyle C₇-C₂₀ substitué ou non, R₄CO- et R₄COO-, R₄ étant un groupement alkyle C₁-C₁₂ linéaire ou ramifié substitué ou non, ou arylalkyle C₇-C₂₀ substitué ou non, parmi les substitutions il faut citer OH, NH₂ ou COOH,
X₁ et X₂ sont des liaisons peptidiques ou pseudopeptidiques,
X₃ est un radical choisi parmi -CO- et -CH₂-, et
R₃ est un groupement choisi parmi -OH, -NH₂, alcoxy C₁-C₁₂ linéaire ou ramifié ou -NH-X₄-CH₂-Z, X₄ étant un groupement hydrocarboné C₁-C₁₂ linéaire ou ramifié, et Z étant un atome d'hydrogène ou un groupement -OH, - CO₂H ou -CONH₂, ainsi que de leurs sels physiologiquement acceptables, dans une composition, en tant qu'agent pour lutter contre la chute des cheveux.

Parmi les groupements alkyles particulièrement adaptés à la mise en oeuvre de la présente invention, on préfèrera les groupements alkyles C₁-C₆ linéaires ou ramifiés. On peut citer plus particulièrement les groupements méthyle, éthyle, propyle, isopropyle, butyle, isobutyle ou tertbutyle.

Par groupement aryle au sens de la présente invention, on entend un groupement carboné aromatique de 6 à 14 atomes de carbone. On peut citer, à titre d'exemple, les groupements phényle, naphtyle ou anthracényle.

Parmi les groupements arylalkyles préférés de l'invention on peut citer les groupements benzyle, et phénéthyle.

Les peptides ou pseudopeptides correspondant à la formule (I) sont dérivés de la structure de base du tétrapeptide Acétyl-Ser-Asp-Lys-Pro (AcSDKP).

Par « radical correspondant à » il faut entendre le radical A de la formule : NH₂-CH(A)-COOH correspondant à l'acide aminé.
Ainsi, A est

-CH₂OH pour Ser,

-CH₂COOH pour Asp,

-CH₂-CH₂-COOH pour Glu,

-(CH₂)₃-NH-C(NH)NH₂ pour Arg,

-(CH₂)₃-NH₂ pour Orn et

-(CH₂)₄-NH₂ pour Lys,

pour l'acide aminé terminal A₄, il s'agit soit de la structure :

=N-CH(A)-CO- ou NH-(CH)A-CO-.

Par « pseudopeptide » on entend désigner des composés similaires aux peptides de référence mais dans lesquels une ou plusieurs liaisons peptidiques - CO-NH- ont été remplacées par une liaison équivalente à la liaison peptidique qui est appelée pseudopeptidique, soit -CH₂-NH-, -CH₂-S-, -CH₂-O-, -CO-CH₂-, - CH₂-CO-, -CH₂-CH₂- représenté par Ψ (CH₂NH) par exemple.

Parmi les radicaux R₁ et R₂, on préférera tout particulièrement l'atome d'hydrogène, ou les radicaux R₄CO- pour lesquels R₄ représente un groupement alkyle C₁-C₃, notamment CH₃CO ainsi que HOOC-CH₂-CH₂-CO-O. De même, R₃ est de préférence NH₂, OH ou NHCH₃.

Parmi les composés de formule (I) convenant à la mise en oeuvre de l'invention, il faut citer :

CH₃CO-Ser-Asp-Lys-Pro-OH

CH₃CO-Ser-Ψ-(CH₂NH)-Asp-Lys-Pro-OH

CH₃CO-Ser-Asp-Ψ-(CH₂NH)-Lys-Pro-OH

CH₃CO-Ser-Asp-Lys-Ψ-(CH₂N)-Pro-OH

CH₃CO-Ser-Ψ-(CH₂NH)-Asp-Lys-Pro-NH₂

CH₃CO-Ser-Asp-Ψ-(CH₂NH)-Lys-Pro-NH₂

CH₃CO-Ser-Asp-Lys-Ψ-(CH₂N)- Pro-NH₂

H-Ser-Ψ-(CH₂NH)-Asp-Lys-Pro-OH

H-Ser-Asp-Ψ-(CH₂NH)-Lys-Pro-OH

H-Ser-Asp-Lys-Ψ-(CH₂N)-Pro-OH

HOOCCH₂CH₂CO-Ser-Ψ-(CH₂NH)-Asp-Lys-Pro-OH

HOOCCH₂CH₂CO-Ser-Asp-Ψ-(CH₂NH)-Lys-Pro-OH

HOOCCH₂CH₂CO-Ser-Asp-Lys-Ψ-(CH₂N)-Pro-OH

H-Ser-Ψ-(CH₂NH)-Asp-Lys-Pro-NH₂

H-Ser-Asp-Ψ-(CH₂NH)-Lys-Pro-NH₂

H-Ser-Asp-Lys-Ψ-(CH₂N)-Pro-NH₂

HOOCCH₂CH₂CO-Ser-Ψ-(CH₂NH)-Asp-Lys-Pro-NH₂

HOOCCH₂CH₂CO-Ser-Asp-Ψ-(CH₂NH)-Lys-Pro-NH₂

HOOCCH₂CH₂CO-Ser-Asp-Lys-Ψ-(CH₂N)-Pro-NH₂

CH₃CO-Ser-Asp-Lys-Pro-NH₂

H-Ser-Asp-Lys-Pro-NH₂

CH₃CO-Ser-Asp-Lys-Pro-NHCH₃

H-Ser-Asp-Lys-Pro-NHCH₃

HOOCCH₂CH₂CO-Ser-Asp-Lys-Pro-NHCH₃

HOOCCH₂CH₂CO-Ser-Asp-Lys-Pro-NH₂.

Un composé de formule (I) convenant particulièrement bien à la mise en oeuvre de la présente invention est le tétrapeptide naturel CH ₃CO-Ser-Asp-Lys-Pro (AcSDKP).

Par le terme « de sel physiologiquement acceptable », on entend au sens de la présente invention tout sel préparé à partir de tout acide non toxique physiologiquement acceptable, y compris les acides organiques et inorganiques. De tels acides incluent l'acide acétique, benzènesulfonique, benzoïque, citrique, éthanesulfonique, fumarique, gluconique, glutamique, bromhydrique, chlorhydrique, lactique, maléique, malique, mandélique, méthanesulfonique, mucique, nitrique, pamoique, pantothénique, phosphorique, succinique, tartarique et paratoluènesulfonique. Avantageusement, on utilise l'acide chlorhydrique.

Les composés de formule (I) telle que définie ci-dessus ont montré une activité stimulante sur la croissance cellulaire et la restructuration des gaines épithéliales des follicules pileux qui constituent le siège des cellules souches. Une forte augmentation du nombre de néo-kératinocytes et de cellules indifférenciées dans les structures pilaires des cheveux soumis à l'action du composé de formule (I) a été observée.

La Demanderesse a mis en évidence qu'au niveau moléculaire, l'effet des composés utilisés selon l'invention se traduisait par une augmentation de l'expression du collagène IV et de la laminine 5 sur toute la longueur du cheveu, avec un effet plus important dans sa partie médiane, située entre le bulge et le bulbe.

Un mode de réalisation de l'invention concerne l'utilisation cosmétique d'un composé de formule (I) telle que définie précédemment, dans une composition, en tant qu'agent induisant la prolifération et/ou la différentiation des cellules souches et/ou des néokératinocytes dans les gaines épithéliales des cheveux et/ou stimulant la production du collagène IV et/ou de la laminine 5 dans le follicule pileux.

Plus particulièrement, la présente invention concerne l'utilisation cosmétique d'un composé de formule (I) telle que définie précédemment, dans une composition, en tant qu'agent pour induire et/ou stimuler la croissance des cheveux.

Dans un autre aspect de l'invention, les composés de formule (I) telle que définie précédemment peuvent être utilisés pour augmenter la densité des cheveux.

Un autre aspect particulier de l'invention concerne l'utilisation des composés de formule (I) telle que définie précédemment pour lutter contre l'alopécie.

Le tétrapeptide naturel CH₃CO-Ser-Asp-Lys-Pro (AcSDKP) (WO-88/00594) peut être obtenu par une synthèse de type peptidique classique. Les peptides ou pseudopeptides de formule (I) apparentés au dérivé AcSDKP peuvent également être obtenus par une synthèse peptidique ou pseudopeptidique, comme décrit dans le document WO-97/28183.

Les composés de formule (I) sont présents dans les compositions utilisées pour la mise en oeuvre de la présente invention en une quantité comprise entre 0,001 % et 10 % en poids de préférence entre 0,005 % et 5 % en poids par rapport au poids total de la composition.

Les compositions cosmétiques utilisées selon l'invention sont destinées à un usage topique, et peuvent se présenter sous toutes les formes galéniques classiquement utilisées pour ce type d'application et notamment sous forme d'émulsions (huile-dans-eau, eau-dans-huile, émulsion triple huile-dans-eau-dans huile, ou eau-dans-huile-dans-eau), de gels aqueux, de solutions aqueuses, hydroalcooliques, ou huileuses. Elles peuvent être plus ou moins fluides et se présenter sous la forme d'une crème blanche ou colorée, d'une pommade, d'un lait, d'une lotion, d'un sérum, d'une pâte, d'une mousse ou d'une biphase. Elles peuvent aussi se présenter sous forme d'aérosol. Les compositions utilisées pour la mise en oeuvre de la présente invention peuvent notamment se présenter sous forme de lotion, de gel, de savon, d'aérosol, de shampoing ou de mousse.

Les compositions utilisées dans le cadre de la présente invention contiennent, outre les dérivés de formule (I), un ou plusieurs excipients qui peuvent être choisis parmi des composés présentant une bonne compatibilité avec les actifs présents dans la formule. Il s'agit par exemple des polymères hydrosolubles de type polymère naturel, tels les polysaccharides (gomme xanthane, gomme de caroube, peptine...) ou polypeptides, des dérivés cellulosiques type méthylcellulose, hydroxypropylcellulose, hydroxypropyl-méthylcellulose ou encore des polymères synthétiques, polaxamères, carbomères, PVA ou PVP.

Enfin, les compositions de la présente invention peuvent contenir également divers autres excipients de type cosolvant comme l'éthanol, le glycérol, l'alcool benzylique, des humectants (glycérol), des agents facilitant la diffusion (transcurol, urée), ou encore des conservateurs anti-bactériens (p-hydroxybenzoate de méthyle à 0,15%). Elles peuvent également contenir des agents tensioactifs, des agents stabilisants, des émulsifiants, des épaississants, d'autres principes actifs conduisant à un effet complémentaire ou éventuellement synergique, des oligo-éléments, des huiles essentielles, des parfums, des colorants, du collagène, des filtres chimiques ou minéraux, des agents hydratants ou des eaux thermales.

Dans un mode de réalisation particulière de l'invention, les dérivés de formule (I) sont combinés avec au moins un autre principe actif.

La présente invention concerne également un procédé cosmétique de traitement et/ou de prévention de la chute des cheveux, en particulier de l'alopécie, comprenant l'application sur le cuir chevelu d'une composition contenant au moins un dérivé de formule (I) telle que définie précédemment.

Les exemples qui suivent servent à illustrer l'invention sans toutefois présenter un caractère limitatif.

### Exemple 1 : Etude Expérimentale

Etude de l'activité stimulante du tétrapeptide AcSDKP sur la croissance de cheveux isolés, maintenus ex vivo en survie. Cette étude a été réalisée sur 66 explants de cheveux humains obtenus à partir d'une plastie de cuir chevelu.

### ■ Mode opératoire

- Les cheveux comprenant les follicules pileux ont été isolés par microdissection et placés individuellement dans des puits (plaque de culture de 96 puits) et maintenus en survie pendant 15 jours dans des conditions de culture cellulaire classiques (37°C, 5% CO₂) dans du milieu de Williams.
- Le tétrapeptide AcSDKP ajouté dans le milieu de culture a été testé aux trois concentrations : 10⁻⁴ M, 10⁻⁷ M, 10⁻¹⁰ M. Le Minoxidil^{®} (2,4-diamino 6-pipéridinopyrimidine 3-oxyde) a été utilisé comme contrôle positif à la concentration 10⁻⁵ M.
- Les milieux de culture contenant les composés testés ont été renouvelés tous les deux jours.
- Les cheveux ont été photographiés à l'aide d'un microscope et d'une caméra CCD couplée à un logiciel d'acquisition et d'archivage.
- A JO, J3, J6, J8, J10, J13 et J15, chaque cheveu a été mesuré à l'aide du module de mesure du logiciel LEICA IM1000 donnant des mesures en µm.
- Aux J8 et J15 du traitement, six cheveux de chaque lot (Témoin non traité, Minoxidil®, + AcSDKP 10⁻⁴ M, + AcSDKP 10⁻⁷ M, + AcSDKP 10⁻¹⁰ M) ont été prélevés et préparés pour des études histologiques.
- Trois explants de chaque lot ont été fixés dans du Bouin ordinaire et les trois autres ont été congelés et conservés à -80°C.

En plus de la mesure de la longueur de chaque cheveu, la morphologie générale des cheveux a été analysée et l'expression de la laminine 5 et du collagène IV, deux composants de la membrane basale, évaluée. De plus, un immunomarquage des cellules en mitoses avec un anticorps anti-Ki67 (protéine nucléaire exprimée dans les cellules en prolifération), a été réalisé afin d'établir un index mitotique des structures folliculaires.

### ■ Résultats

### 1. Taille des cheveux

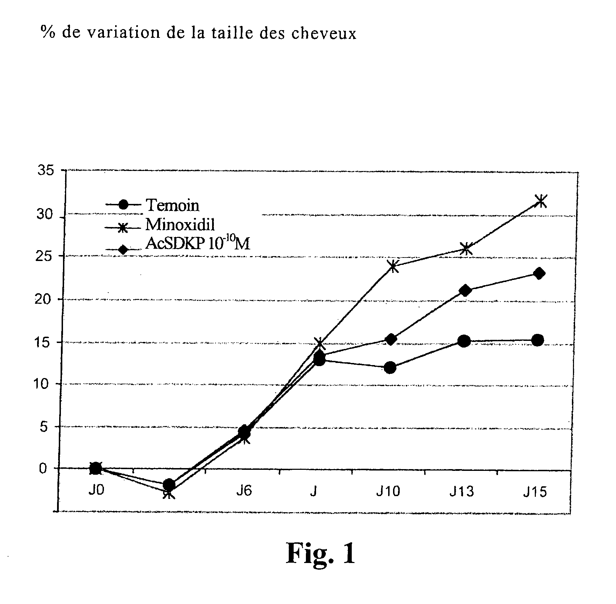

Chaque cheveu a été photographié et sa longueur a été évaluée à l'aide du logiciel dormant des valeurs en µm. Les observations réalisées à J15 de traitement sur l'ensemble des explants montrent que la présence de AcSDKP à 10⁻¹⁰ M dans le milieu de culture stimule significativement la pousse des cheveux (Fig. 1). En revanche, aucune différence significative dans la longueur des tiges pilaires n'a été mise en évidence en présence de AcSDKP testé aux concentrations plus fortes.

### 2. Prolifération cellulaire

Cette étude a été réalisée sur les coupes des cheveux congelés. L'immunomarquage, des cellules présentes sur toute la longueur des gaines épithéliales avec l'anticorps anti-Ki67, met en évidence l'activité mitogène de AcSDKP. En effet, les résultats présentés dans le Tableau 1 montrent que AcSDKP, aux trois concentrations testées, augmente significativement le nombre de cellules en mitose dans les cheveux traités par rapport aux cheveux témoins. Les mesures faites à J8 du traitement montrent que cet effet est maximal pour AcSDKP a 10⁻¹⁰ M (+300 %). L'évaluation du nombre de cellules en mitose indique également que l'activité stimulante de AcSDKP est significativement supérieure à celle visualisée avec le Minoxidil^{®} à 10⁻⁵ M (+38 %) surtout à J8 du traitement.
Les observations réalisées sur l'ensemble des cheveux montrent donc que AcSDKP induit *ex vivo*, de façon plus rapide que le Minoxidil^{®}, une prolifération cellulaire au niveau des gaines épithéliales externes, régions dans lesquelles sont notamment localisées les cellules souches lorsque le cheveu est en phase de croissance.

**Tableau 1. Nombre moyen de cellules en mitose par mm de cheveu**

| Jours | Témoin | Minoxidil^{®} *10⁻⁵ M* | AcSDKP *10⁻⁴ M* | AcSDKP *10⁻⁷ M* | AcSDKP *10⁻¹⁰ M* |
|---|---|---|---|---|---|
| J0 | 12.6 | | | | |
| J8 | 13.4 | 18.5 | 35.4 | 34 | 52.6 |
| J15 | 24.5 | 23.3 | 23.4 | 21.5 | 29.3 |

### 3. Morphologie générale

L'analyse de la morphologie générale des coupes histologiques colorées au trichrome de Masson a été centrée sur la zone comprise depuis l'abouchement des glandes sébacées jusqu'au bulbe du cheveu.

La structure du cheveu mis en culture à J0 s'altère naturellement durant la survie comme cela est observé après une greffe *in vivo*. L'activité restructurante de AcSDKP a notamment été visualisée par l'apparition de néokératinocytes, le long de la gaine conjonctive, qui migrent depuis le bulge, et plus modérément depuis le bulbe, deux régions où résident les cellules souches. Les observations réalisées sur l'ensemble des cheveux isolés montrent que la présence de AcSDKP dans le milieu de survie induit à partir du J8 du traitement une restructuration des gaines épithéliales. Cet effet se traduit par une nette apparition des néo-kératinocytes sur toute la longueur du cheveu depuis l'épiderme jusqu'au bulbe. La structure des néo-kératinocytes formés est homogène et stratifiée. Elle est différente en fonction du temps du traitement et de la concentration de AcSDKP. La plus nette activité a été mise en évidence au J15 du traitement avec AcSDKP à 10⁻¹⁰ M.

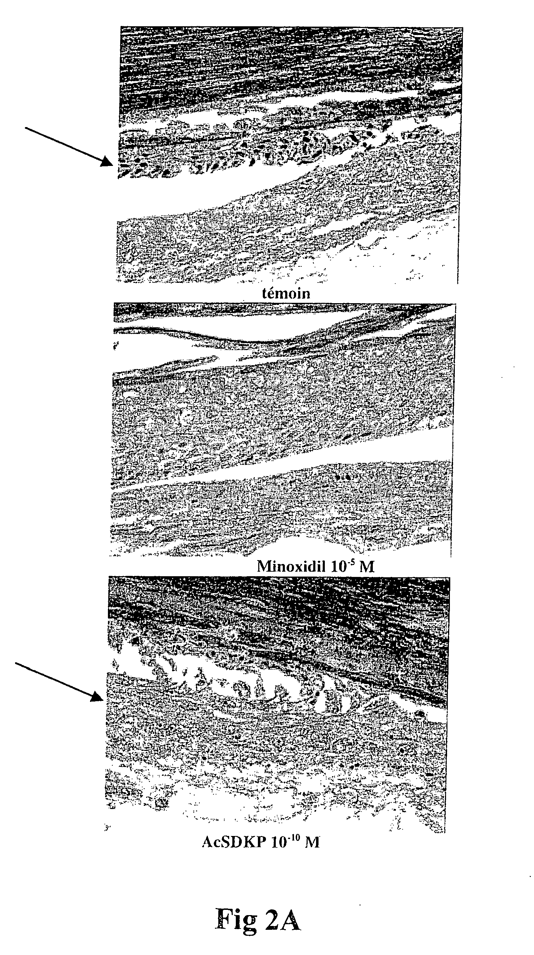

A J8, 2 à 3 assises de néo-kératinocytes ont été visualisées dans les cheveux témoins alors qu'elles sont absentes dans les explants traités avec le Minoxidil^{®} à 10 µM ainsi qu'avec AcSDKP à 10⁻⁴ M. En revanche, une très nette apparition de néo-kératinocytes (5 a 6 assises cellulaires) a été mise en évidence sur toute la longueur des cheveux traités avec AcSDKP à 10⁻⁷ M et 10⁻¹⁰ M. II faut souligner que cet épithélium est parfaitement adhérent au collagène sous-jacent dans les cheveux maintenus en survie en présence de AcSDKP testé aux deux concentrations actives, alors que les gaines épithéliales sont plus ou moins décollées du collagène chez les témoins ainsi qu'en présence de AcSDKP à 10⁻⁴ M et du Minoxidil^{®} (Fig. 2A).

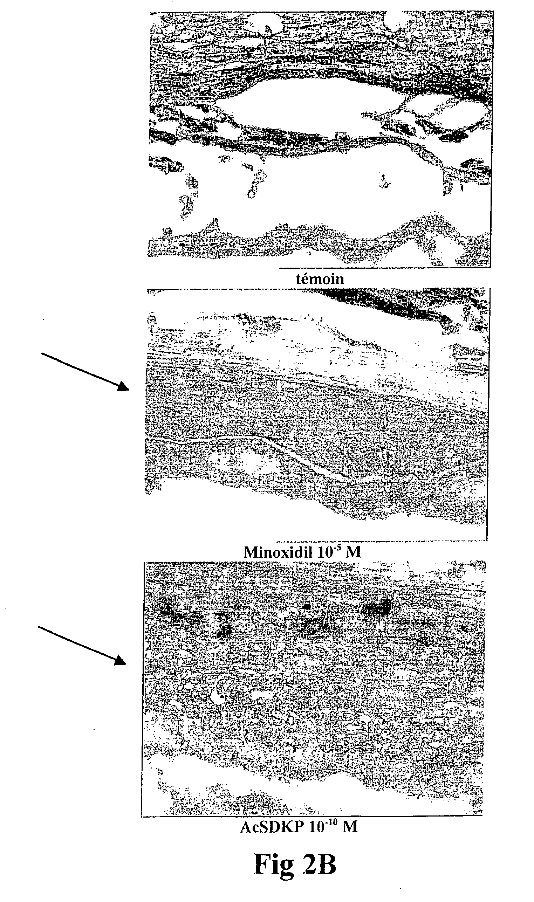

A J15, dans les cheveux traités, les néo-kératinocytes forment une structure homogène, et modérément stratifiée. Elle est légèrement mieux structurée avec le Minoxidil^{®} à 10⁻⁵ M par rapport aux cheveux non traités. Elle devient de plus en plus épaisse avec AcSDKP à 10⁻⁴ M, 10⁻⁷ M et surtout 10⁻¹⁰ M avec 6 à 7 assises cellulaires près du bulbe et jusqu'à 11 à 12 au niveau du bulge (Fig. 2B).

### 4. Expression du collagène de la membrane basale (type IV)

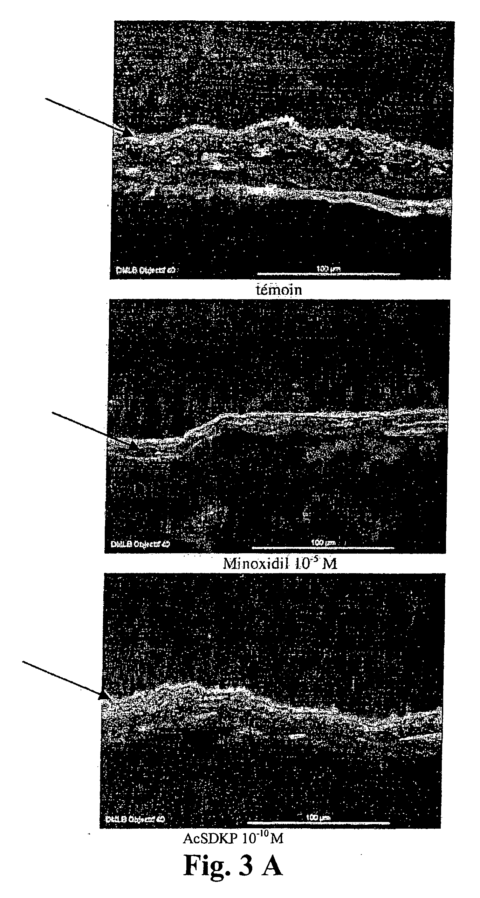

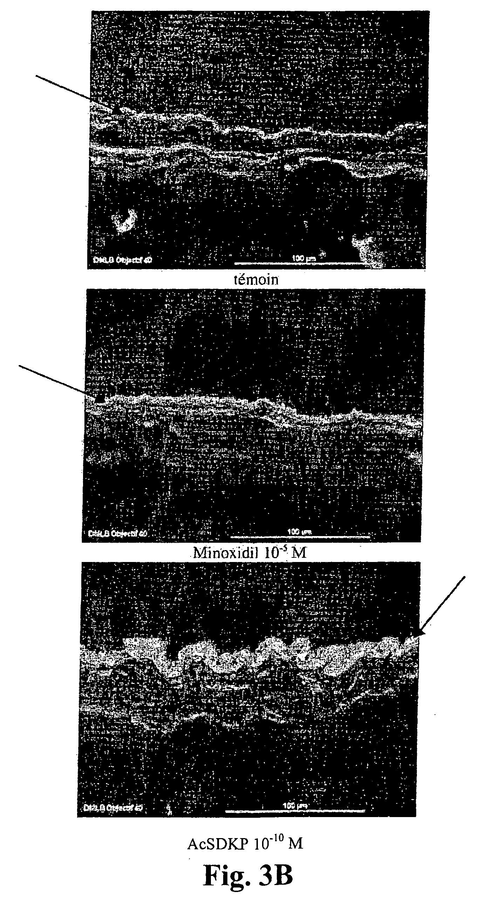

L'immunomarquage du collagène de type IV a été réalisé sur les coupes congelées des cheveux témoins et traités avec AcSDKP ou le Minoxidil^{®} afin de quantifier ce composant de la membrane basale qui sépare le compartiment dermique du compartiment épithélial. L'analyse des résultats obtenus a révélé une activité stimulante de AcSDKP sur l'expression de cette protéine dans les cheveux maintenus en survie en présence du tétrapeptide. En effet, à partir du J8 de traitement avec AcSDKP aux trois concentrations testées, on observe une très nette augmentation du collagène IV au niveau du bulbe et surtout dans la partie médiane des cheveux située entre le bulge et le bulbe. L'augmentation du taux du collagène visualisée aux J8 et J15 est plus importante pour AcSDKP testé à la concentration 10⁻¹⁰ M. Cette surexpression du collagène IV est souvent plus modérée dans les cheveux soumis au traitement avec du Midoxidil^{®} (Fig. 3A et Fig. 3B).

### 5. Expression de la laminine 5

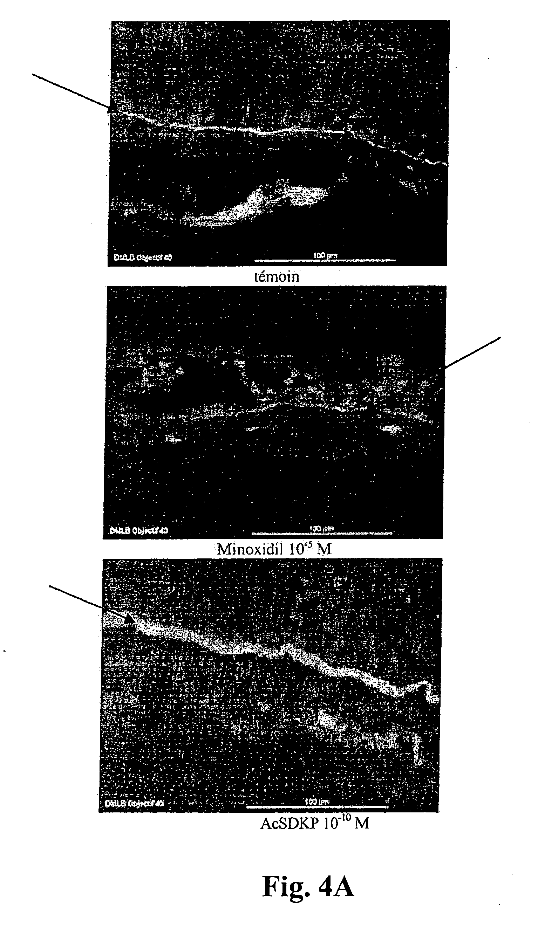

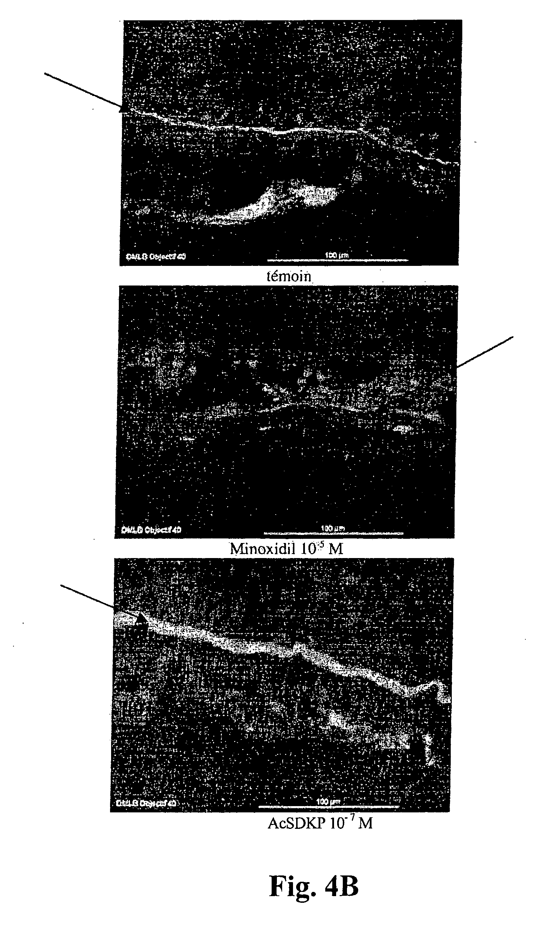

La laminine 5, un autre composant de la membrane basale, est présente dans le bulbe tout autour de la papille dermique ainsi que dans la partie médiane des cheveux. L'immunomarquage de la laminine 5 a permis de mettre en évidence une augmentation significative du taux de cette protéine dans la structure pilaire suite à l'exposition des cheveux au AcSDKP. Cet effet est plus nettement visualisé dans la zone médiane des cheveux traités avec AcSDKP testé à la concentration 10⁻¹⁰ M (Fig. 4A) au J8 du traitement. Cependant au J15, l'expression de la laminine 5 est plus importante pour AcSDKP à 10⁻⁷ M (Fig. 4B).

### 6. Expression de la kératine 19 (CK19), un marqueur des cellules souches épithéliales

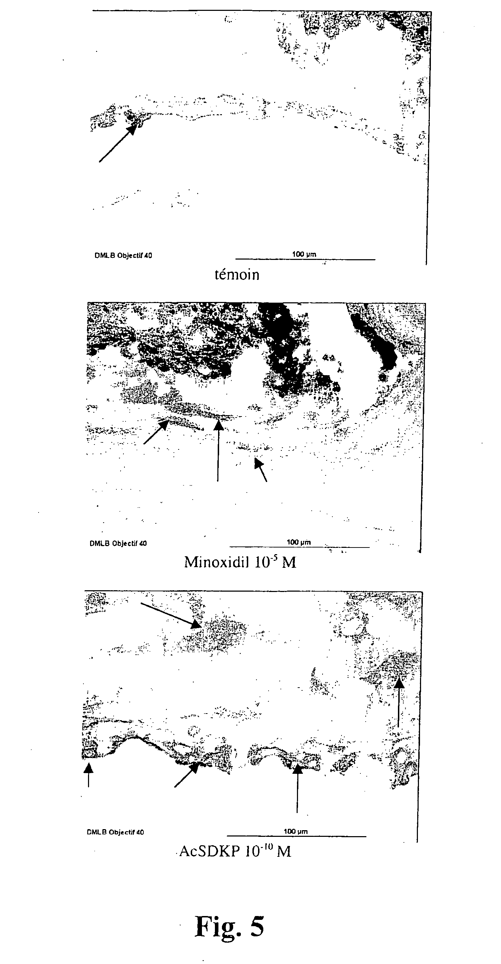

L'immunomarquage de la kératine 19 (CK19) a été réalisé sur les coupes congelées des cheveux témoins et traités avec AcSDKP ou le Minoxidil^{®} afin d'évaluer la proportion des cellules primitives indifférenciées (voir cellules souches) présentes dans les gaines épithéliales externes des follicules pileux. Les résultats obtenus montrent que l'expression de la CK19 est significativement plus forte dans les cheveux maintenus en survie en présence du tétrapeptide par rapport aux cheveux contrôles et surtout par rapport aux cheveux soumis à l'action du Minoxidil^{®}. L'augmentation du taux de la CK19 aux J8 et J15 du traitement est maximale pour AcSDKP testé à la concentration 10⁻¹⁰ M (Fig. 5). L'analyse des résultats obtenus a révélé une activité stimulante de AcSDKP au niveau des cellules souches épithéliales réparties le long de la gaine externe du follicule et responsables du renouvellement cyclique du follicule.

### 7. Localisation tissulaire de AcSDKP radiomarqué chez l'animal ayant reçu in vivo une dose unique du tétrapeptide.

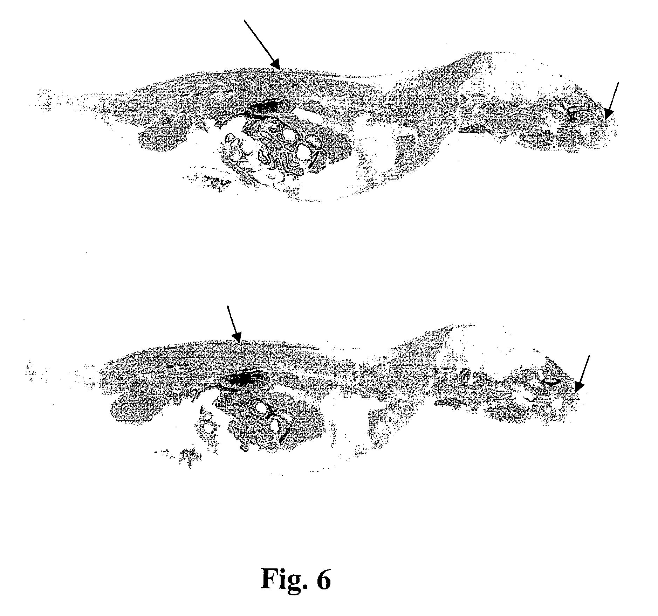

De façon complémentaire, une approche par marquage *in vivo* des organes cibles du peptide a été réalisée. Cette étude a été développée a l'aide de [³H]AcSDKP (100 Ci/mmole) spécifiquement tritié dans la chaîne latérale de la lysine. L'injection in vivo de [³H]AcSDKP chez le rat, suivie d'une autoradiographie de coupes réalisées sur l'animal entier a notamment permis de visualiser l'accumulation de la molécule dans la peau de l'animal ainsi que dans les follicules pileux des moustaches (Fig. 6). Cette localisation préférentielle de AcSDKP suggère que l'activité biologique de ce tétrapeptide s'exerce probablement au niveau de ces régions.

### Exemple 2 : Lotion

| | |
|---|---|
| Alcool éthylique | 50 % |
| AcSDKP | 0,1 % |
| Eau | 49,9 % |
| Parfum q.s. | |

### Exemple 3 : Lotion

| | |
|---|---|
| Hydroxyéthylcellulose | 0,4 % |
| Alcool éthylique | 25 % |
| Butane 1,3-diol | 38,4 % |
| Paraméthylbenzoate | 0,2 % |
| AcSDKP | 0,05 % |
| Parfum | 1 % |
| Eau q.s.p | 100 % |

### Exemple 4 : Emulsion Eau-dans-Huile

| **Phase huileuse** | | |
|---|---|---|
| Vitamine E | | 1 % |
| Monoléate de sorbitane | | 2 % |
| Quaternum -18 | | 0,5 % |
| Paraffine | | 6,5 % |

| **Phase aqueuse** | | |
|---|---|---|
| AcSDKP | | 0,5 % |
| Gomme xanthane | | 1 % |
| Conservateur | | 0,3 % |
| Parfum | q.s. | |
| Eau | q.s.p | 100 % |

## Revendications

1. Utilisation cosmétique d'au moins un composé de formule (I) : 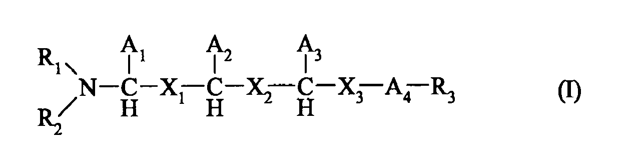 dans laquelle,
A₁ est le radical correspondant à D- ou L- Ser,
A₂ est le radical correspondant à D- ou L- Asp ou Glu,
A₃ est le radical correspondant à D- ou L- Lys, Arg ou Orn,
A₄ est le radical correspondant à D- ou L- Pro,
R₁ et R₂ sont choisis indépendamment parmi l'atome d'hydrogène, les groupements alkyle C₁-C₁₂ linéaire ou ramifié substitué ou non, arylalkyle C₇-C₂₀ substitué ou non, R₄CO- et R₄COO-, R₄ étant un groupement alkyle C₁-C₁₂ linéaire ou ramifié substitué ou non, ou arylalkyle C₇-C₂₀ substitué ou non, parmi les substitutions il faut citer OH, NH₂ ou COOH,
X₁ et X₂ sont des liaisons peptidiques ou pseudopeptidiques,
X₃ est un radical choisi parmi -CO- et -CH₂-, et
R₃ est un groupement choisi parmi -OH, -NH₂, alcoxy C₁-C₁₂ linéaire ou ramifié ou -NH-X₄-CH₂-Z, X₄ étant un groupement hydrocarboné C₁-C₁₂ linéaire ou ramifié, et Z étant un atome d'hydrogène ou un groupement -OH, - CO₂H ou -CONH₂,
ainsi que de leurs sels physiologiquement acceptables, dans une composition, en tant qu'agent pour lutter contre la chute des cheveux.

2. Utilisation cosmétique d'un composé de formule (I) telle que définie dans la revendication 1, dans une composition, en tant qu'agent stimulant la prolifération et/ou la différentiation des cellules souches et/ou des néokératinocytes dans les gaines épithéliales des cheveux.

3. Utilisation cosmétique d'un composé de formule (I) telle que définie dans la revendication 1, dans une composition, en tant qu'agent stimulant la production du collagène IV et/ou de la laminine 5 dans le follicule pileux.

4. Utilisation cosmétique d'un composé de formule (I) telle que définie dans la revendication 1, dans une composition, en tant qu'agent pour induire et/ou stimuler la croissance des cheveux.

5. Utilisation selon l'une des revendications 1 à 4, pour augmenter la densité des cheveux.

6. Utilisation selon l'une des revendications 1 à 5, pour lutter contre l'alopécie

7. Utilisation selon l'une des revendications 1 à 6, **caractérisée en ce que** le dérivé de formule (I) comporte au moins une liaison pseudopeptidique.

8. Utilisation selon l'une des revendications 1 à 7, **caractérisée en ce que** le composé de formule (I) est choisi parmi :
CH₃CO-Ser-Asp-Lys-Pro-OH
CH₃CO-Ser-Ψ-(CH₂NH)-Asp-Lys-Pro-OH
CH₃CO-Ser-Asp-Ψ-(CH₂NH)-Lys-Pro-OH
CH₃CO-Ser-Asp-Lys-Ψ-(CH_{2N})-Pro-OH
CH₃CO-Ser-Ψ-(CH₂NH)-Asp-Lys-Pro-NH₂
CH₃CO-Ser-Asp-Ψ-(CH₂NH)-Lys-Pro-NH₂
CH₃CO-Ser-Asp-Lys-Ψ-(CH₂N)- Pro-NH₂
H-Ser-Ψ-(CH₂NH)-Asp-Lys-Pro-OH
H-Ser-Asp-Ψ-(CH₂NH)-Lys-Pro-OH
H-Ser-Asp-Lys-Ψ-(CH₂N)-Pro-OH
HOOCCH₂CH₂CO-Ser-Ψ-(CH₂NH)-Asp-Lys-Pro-OH
HOOCCH₂CH₂CO-Ser-Asp-Ψ-(CH₂NH)-Lys-Pro-OH
HOOCCH₂CH₂CO-Ser-Asp-Lys-Ψ-(CH₂N)-Pro-OH
H-Ser-Ψ-(CH₂NH)-Asp-Lys-Pro-NH₂
H-Ser-Asp-Ψ-(CH₂NH)-Lys-Pro-NH₂
H-Ser-Asp-Lys-Ψ-(CH₂N)-Pro-NH₂
HOOCCH₂CH₂CO-Ser-Ψ-(CH₂NH)-Asp-Lys-Pro-NH₂
HOOCCH₂CH₂CO-Ser-Asp-Ψ-(CH₂NH)-Lys-Pro-NH₂
HOOCCH₂CH₂CO-Ser-Asp-Lys-Ψ-(CH₂N)-Pro-NH₂
CH₃CO-Ser-Asp-Lys-Pro-NH₂
H-Ser-Asp-Lys-Pro-NH₂
CH₃CO-Ser-Asp-Lys-Pro-NHCH₃
H-Ser-Asp-Lys-Pro-NHCH₃
HOOCCH₂CH₂CO-Ser-Asp-Lys-Pro-NHCH₃
HOOCCH₂CH₂CO-Ser-Asp-Lys-Pro-NH₂.

9. Utilisation selon l'une des revendications 1 à 8, **caractérisée en ce que** le composé de formule (I) est représenté par la formule : CH₃CO-Ser-Asp-Lys-Pro-OH.

10. Utilisation selon l'une des revendications 1 à 9, **caractérisée en ce que** le composé de formule (I) est présent dans la composition en une quantité comprise entre 0,001 % et 10 % en poids par rapport au poids total de la composition.

11. Procédé cosmétique de traitement et/ou de prévention de la chute des cheveux, en particulier de l'alopécie, comprenant l'application sur le cuir chevelu d'une composition contenant au moins un dérivé de formule (I) telle que définie dans la revendication 1.

## Claims

1. Cosmetic use of at least one compound of formula (I) : 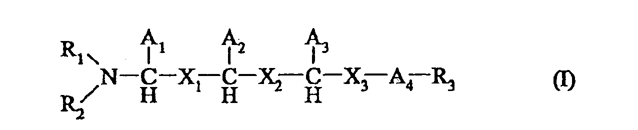 in which
A₁ is the radical corresponding to D- or L-Ser,
A₂ is the radical corresponding to D- or L-Asp or Glu,
A₃ is the radical corresponding to D- or L-Lys, Arg or Orn,
A₄ is the radical corresponding to D- or L-Pro,
R₁ and R₂ are independently chosen from among the hydrogen atom, linear or branched substituted or non-substituted C₁-C₁₂ alkyl groups, substituted or non-substituted C₇-C₂₀ arylalkyl groups, R₄CO- and R₄COO-, where R₄ is a linear or branched substituted or non-substituted C₁-C₁₂ alkyl group, or a substituted or non-substituted C₇-C₂₀ arylalkyl, where substitutions include OH, NH₂ and COOH,
X₁ and X₂ are peptide or pseudopeptide bonds,
X₃ is a radical chosen from among -CO- and -CH₂-, and
R₃ is a group chosen from among -OH, -NH₂, linear or branched alcoxy C₁-C₁₂ or -NH-X₄-CH₂-Z, where X₄ is a linear or branched C₁-C₁₂ hydrocarbon group and Z is a hydrogen atom or -OH, -CO₂H or -CONH₂,
as well as their physiologically acceptable salts in a composition as an agent for controlling hair loss.

2. Cosmetic use of a compound of formula (I) as defined in claim 1, in a composition, as an agent to stimulate the proliferation and/or differentiation of stem cells and/or neo-keratinocytes in the epithelial sheath of hair.

3. Cosmetic use of a compound of formula (I) as defined in claim 1, in a composition, as an agent to stimulate the production of collagen IV and/or laminin 5 in the hair follicle.

4. Cosmetic use of a compound of formula (I) as defined in claim 1, in a composition, as an agent to trigger and/or stimulate hair growth.

5. Use according to one of claims 1 to 4 to increase hair density.

6. Use according to one of claims 1 to 5 to control alopecia.

7. Use according to one of claims 1 to 6 **characterized in that** the derivative of formula (I) includes at least one pseudopeptide bond.

8. Use according to one of claims 1 to 7 **characterized in that** the compound of formula (I) is chosen from among:
CH₃CO-Ser-Asp-Lys-Pro-OH,
CH₃CO-Ser-Ψ-(CH₂NH)-Asp-Lys-Pro-OH
CH₃CO-Ser-Asp-Ψ-(CH₂NH)-Lys-Pro-OH
CH₃CO-Ser-Asp-Lys-Ψ-(CH₂N)-Pro-OH
CH₃CO-Ser-Ψ-(CH₂NH)-Asp-Lys-Pro-NH₂
CH₃CO-Ser-Asp-Ψ-(CH₂NH)-Lys-Pro-NH₂
CH₃CO-Ser-Asp-Lys-Ψ-(CH₂N)-Pro-NH₂
H-Ser-Ψ-(CH₂NH)-Asp-Lys-Pro-OH
H-Ser-Asp-Ψ-(CH₂NH)-Lys-Pro-OH
H-Ser-Asp-Lys-Ψ-(CH₂N)-Pro-OH
HOOCCH₂CH₂CO-Ser-Ψ-(CH₂NH)-Asp-Lys-Pro-OH
HOOCCH₂CH₂CO-Ser-Asp-Ψ-(CH₂NH)-Lys-Pro-OH
HOOCCH₂CH₂CO-Ser-Asp-Lys-Ψ-(CH₂N)-Pro-OH
H-Ser-Ψ-(CH₂NH)-Asp-Lys-Pro-NH₂
H-Ser-Asp-Ψ-(CH₂NH)-Lys-Pro-NH₂
H-Ser-Asp-Lys-Ψ-(CH₂N)-Pro-NH₂
HOOCCH₂CH₂CO-Ser-Ψ-(CH₂NH)-Asp-Lys-Pro-NH₂
HOOCCH₂CH₂CO-Ser-Asp-Ψ-(CH₂NH)-Lys-Pro-NH₂
HOOCCH₂CH₂CO-Ser-Asp-Lys-Ψ-(CH₂N)-Pro-NH₂
CH₃CO-Ser-Asp-Lys-Pro-NH₂
H-Ser-Asp-Lys-Pro-NH₂
CH₃CO-Ser-Asp-Lys-Pro-NHCH₃
H-Ser-Asp-Lys-Pro-NHCH₃
HOOCCH₂CH₂CO-Ser-Asp-Lys-Pro-NHCH₃
HOOCCH₂CH₂CO-Ser-Asp-Lys-Pro-NH₂

9. Use according to one of claims 1 to 8, **characterized in that** the compound of formula (I) is represented by the formula: CH₃CO-Ser-Asp-Lys-Pro-OH.

10. Use according to one of claims 1 to 9, **characterized in that** the compound of formula (I) is present in the composition in amounts ranging from 0.001% to 10% by weight of the total weight of the composition.

11. Cosmetic process for the treatment and/or prevention of hair loss, particularly alopecia, consisting of the application to the scalp of a composition containing at least one derivative of formula (I) as defined in claim 1.

## Patentansprüche

1. Kosmetische Verwendung mindestens einer Verbindung der Formel (I): 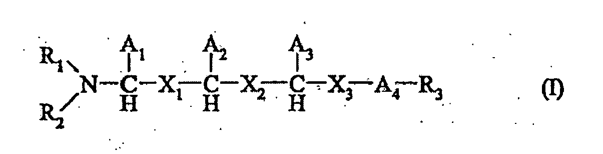 in der
A₁ ein Rest ist, der D- oder L-Ser entspricht,
A₂ ein Rest ist, der D- oder L-Asp oder Glu entspricht,
A₃ ein Rest ist, der D- oder L-Lys, Arg oder Orn entspricht,
A₄ ein Rest ist, der D- oder L-Pro entspricht,
R₁ und R₂ unabhängig aus einem Wasserstoffatom, linearen oder verzweigten, substituierten oder unsubstituierten (C₁-C₁₂)-Alkylgruppen, substituierten oder unsubstituierten (C₇-C₂₀)-Aralkylgruppen, den Gruppen R₄CO- und R₄COO- ausgewählt sind, wobei R₄ eine lineare oder verzweigte, substituierte oder unsubstituierte (C₁-C₁₂)-Alkylgruppe oder eine substituierte oder unsubstituierte (C₇-C₂₀)-Aralkylgruppe ist,
wobei bei den Substitutionen OH, NH₂ oder COOH angeführt werden müssen,
X₁ und X₂ peptidische oder pseudopeptidische Bindungen sind,
X₃ ein Rest ist, der aus -CO- und -CH₂- ausgewählt ist, und
R₃ eine Gruppe ist, die aus -OH, -NH₂, linearem oder verzweigtem (C₁-C₁₂)-Alkoxy oder -NH-X₄-CH₂-Z ausgewählt ist, wobei X₄ eine lineare oder verzweigte (C₁-C₁₂)-Kohlenwasserstoffgruppe ist und Z ein Wasserstoffatom oder eine Gruppe -OH, -CO₂H oder -CONH₂ ist,
sowie von deren physiologisch annehmbaren Salzen in einer Zusammensetzung als Mittel zur Bekämpfung des Haarausfalls.

2. Kosmetische Verwendung einer Verbindung der Formel (I), wie in Anspruch 1 definiert, in einer Zusammensetzung als Mittel, das die Proliferation und/oder die Differenzierung von Stammzellen und/oder Neokeratinozyten in den Epithelschäften der Haare stimuliert.

3. Kosmetische Verwendung einer Verbindung der Formel (I), wie in Anspruch 1 definiert, in einer Zusammensetzung als Mittel zur Stimulierung der Produktion von Kollagen IV und/oder von Laminin 5 im Haarfollikel.

4. Kosmetische Verwendung einer Verbindung der Formel (I), wie in Anspruch 1 definiert, in einer Zusammensetzung als Mittel zur Induktion und/oder Stimulation des Wachstums von Haaren.

5. Verwendung nach einem der Ansprüche 1 bis 4, um die Dichte der Haare zu erhöhen.

6. Verwendung nach einem der Ansprüche 1 bis 5, um Alopezie zu bekämpfen.

7. Verwendung nach einem der Ansprüche 1 bis 6, **dadurch gekennzeichnet, dass** das Derivat der Formel (I) mindestens eine pseudopeptidische Bindung umfasst.

8. Verwendung nach einem der Ansprüche 1 bis 7, **dadurch gekennzeichnet, dass** die Verbindung der Formel (I) ausgewählt ist aus:
CH₃CO-Ser-Asp-Lys-Pro-OH
CH₃CO-Ser-Ψ-(CH₂NH)-Asp-Lys-Pro-OH
CH₃CO-Ser-Asp-Ψ-(CH₂NH)-Lys-Pro-OH
CH₃CO-Ser-Asp-Lys-Ψ-(CH₂N)-Pro-OH
CH₃CO-Ser-Ψ-(CH₂NH)-Asp-Lys-Pro-NH₂
CH₃CO-Ser-Asp-Ψ-(CH₂NH)-Lys-Pro-NH₂
CH₃CO-Ser-Asp-Lys-Ψ-(CH₂N)-Pro-NH₂
H-Ser-Ψ-(CH₂NH)-Asp-Lys-Pro-OH
H-Ser-Asp-Ψ-(CH₂NH)-Lys-Pro-OH
H-Ser-Asp-Lys-Ψ-(CH₂N)-Pro-OH
HOOCCH₂CH₂CO-Ser-Ψ-(CH₂NH)-Asp-Lys-Pro-OH
HOOCCH₂CH₂CO-Ser-Asp-Ψ-(CH₂NH)-Lys-Pro-OH
HOOCCH₂CH₂CO-Ser-Asp-Lys-Ψ-(CH₂N)-Pro-OH
H-Ser-Ψ-(CH₂NH)-Asp-Lys-Pro-NH₂
H-Ser-Asp-Ψ-(CH₂NH)-Lys-Pro-NH₂
H-Ser-Asp-Lys-Ψ-(CH₂N)-Pro-NH₂
HOOCCH₂CH₂CO-Ser-Ψ-(CH₂NH)-Asp-Lys-Pro-NH₂
HOOCCH₂CH₂CO-Ser-Asp-Ψ-(CH₂NH)-Lys-Pro-NH₂
HOOCCH₂CH₂CO-Ser-Asp-Lys-Ψ-(CH₂N)-Pro-NH₂
CH₃CO-Ser-Asp-Lys-Pro-NH₂
H-Ser-Asp-Lys-Pro-NH₂
CH₃CO-Ser-Asp-Lys-Pro-NHCH₃
H-Ser-Asp-Lys-Pro-NHCH₃
HOOCCH₂CH₂CO-Ser-Asp-Lys-Pro-NHCH₃
HOOCCH₂CH₂CO-Ser-Asp-Lys-Pro-NH₂.

9. Verwendung nach einem der Ansprüche 1 bis 8, **dadurch gekennzeichnet**, das die Verbindung der Formel (I) durch die Formel: CH₃CO-Ser-Asp-Lys-Pro-OH dargestellt wird.

10. Verwendung nach einem der Ansprüche 1 bis 9, **dadurch gekennzeichnet, dass** die Verbindung der Formel (I) in der Zusammensetzung in einer Menge vorliegt, die zwischen 0,001 und 10 Gew.-% einschließlich, bezogen auf das Gesamtgewicht der Zusammensetzung, vorliegt.

11. Kosmetisches Verfahren zur Behandlung und/oder Verhütung des Ausfalls von Haaren, insbesondere der Alopezie, umfassend das Auftragen einer Zusammensetzung, die mindestens ein Derivat der in Anspruch 1 definierten Formel (I) enthält, auf die Kopfhaut.
